(19) Europäisches Patentamt
European Patent Office
Office européen des brevets

(11) Veröffentlichungsnummer: **0 414 058 B1**

(12) # EUROPÄISCHE PATENTSCHRIFT

(45) Veröffentlichungstag der Patentschrift: **15.06.94**

(21) Anmeldenummer: **90115343.7**

(22) Anmeldetag: **10.08.90**

(51) Int. Cl.5: **C07D 401/12**, C07D 239/60, C07D 413/12, C07D 251/30, C07D 251/38, C07D 413/14, A01N 43/54, A01N 43/66

(54) **Carbonsäurederivate.**

(30) Priorität: **19.08.89 DE 3927382**

(43) Veröffentlichungstag der Anmeldung:
**27.02.91 Patentblatt 91/09**

(45) Bekanntmachung des Hinweises auf die Patenterteilung:
**15.06.94 Patentblatt 94/24**

(84) Benannte Vertragsstaaten:
**AT BE CH DE ES FR GB IT LI NL**

(56) Entgegenhaltungen:
**EP-A- 0 223 406**
**EP-A- 0 249 707**
**EP-A- 0 346 789**

(73) Patentinhaber: **BASF Aktiengesellschaft**
**Carl-Bosch-Strasse 38**
**D-67063 Ludwigshafen(DE)**

(72) Erfinder: **Rheinheimer, Joachim, Dr.**
**Merziger Strasse 24**
**D-6700 Ludwigshafen(DE)**
Erfinder: **Eicken, Karl, Dr.**
**Am Huettenwingert 12**
**D-6706 Wachenheim(DE)**
Erfinder: **Vogelbacher, Uwe Josef, Dr.**
**Niedererdstrasse 56**
**D-6700 Ludwigshafen(DE)**
Erfinder: **Rohr, Wolfgang, Dr.**
**In der Dreispitz 13**
**D-6706 Wachenheim(DE)**
Erfinder: **Keukenhoehner, Thomas, Dr.**
**Seidelstrasse 2**
**D-6710 Frankenthal(DE)**
Erfinder: **Westphalen, Karl-Otto, Dr.**
**Mausbergweg 58**
**D-6720 Speyer(DE)**
Erfinder: **Wuerzer, Bruno, Dr.**
**Ruedigerstrasse 13**
**D-6701 Otterstadt(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 414 058 B1

**Beschreibung**

Die vorliegende Erfindung betrifft Carbonsäurederivate der allgemeinen Formel I

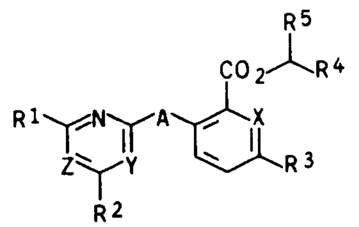

I,

in der die Substituenten folgende Bedeutung haben:
$R^1$, $R^2$
$C_1$-$C_4$-Alkyl, $C_1$-$C_4$-Halogenalkyl, $C_1$-$C_4$-Alkoxy, $C_1$-$C_4$-Halogenalkoxy $C_1$-$C_4$-Alkylthio;
$R^3$
Wasserstoff; Hydroxy; Cyano; Nitro; Amino; Formyl;
Halogen;
eine $C_1$-$C_4$-Alkylgruppe, eine $C_1$-$C_4$-Alkoxygruppe, eine $C_3$-$C_6$-Alkenylgruppe, eine $C_3$-$C_6$-Alkenyloxygruppe, eine $C_3$-$C_6$-Alkinylgruppe oder eine $C_3$-$C_6$-Alkinyloxygruppe, wobei diese Gruppen ein bis fünf Halogenatome und/oder eine $C_1$-$C_4$-Alkoxy- oder $C_1$-$C_4$-Alkylthiogruppe tragen können;
eine $C_1$-$C_4$-Alkylaminogruppe; eine Di-$C_1$-$C_4$-Alkylaminogruppe; eine Phenylaminogruppe; eine N-Phenyl-N-$C_1$-$C_4$-alkylaminogruppe oder eine $C_1$-$C_6$-Alkylcarbonylaminogruppe;
$R^4$
Wasserstoff oder $C_1$-$C_4$-Alkyl;
$R^5$
einen fünfgliedrigen Heteroarylrest, der ein oder zwei Stickstoffatome und ein Sauerstoff- oder Schwefelatom enthält, oder einen Isoxazolinylrest, wobei diese Ringsysteme ein bis vier Halogenatome und/oder ein oder zwei der folgenden Reste tragen können: $C_1$-$C_6$-Alkyl, $C_1$-$C_2$-Halogenalkyl, $C_1$-$C_4$-Alkoxy, $C_1$-$C_2$-Halogenalkoxy, $C_3$-$C_8$-Cycloalkyl, Phenyl oder Pyridyl, wobei die aromatischen Reste ihrerseits ein bis fünf Halogenatome und/oder ein bis drei der bei $R^1$ genannten Reste tragen können;
oder eine Gruppe -$CR^6$ = $NOR^7$, worin
$R^6$
Wasserstoff;
$C_1$-$C_4$-Alkyl, bedeutet welches ein bis fünf Halogenatome und/oder einen der folgenden Reste tragen kann: $C_1$-$C_4$-Alkoxy, $C_1$-$C_4$-Alkylthio oder Phenyl, wobei der Phenylrest seinerseits ein bis fünf Halogenatome und/oder ein bis drei der bei $R^1$ genannten Reste tragen kann, oder für $C_3$-$C_8$-Cycloalkyl steht, welches ein bis drei $C_1$-$C_4$-Alkylgruppen tragen kann,
Phenyl, welches ein bis fünf Halogenatome und/oder ein bis drei der bei $R^1$ genannten Reste tragen kann, bedeutet und
$R^7$
$C_1$-$C_8$-Alkyl oder $C_3$-$c_6$-Alkenyl, welches ein bis fünf Halogenatome und/oder einen der folgenden Reste tragen kann: $C_1$-$C_4$-Alkoxy, $C_1$-$C_4$-Alkylthio oder Phenyl, wobei der Phenylrest seinerseits ein bis fünf Halogenatome und/oder ein bis drei der bei $R^1$ genannten Reste tragen kann;
$C_3$-$C_8$-Cycloalkyl, welches ein bis drei $C_1$-$C_4$-Alkylgruppen tragen kann; Phenyl, welches ein bis fünf Halogenatome und/oder ein bis drei der bei $R^1$ genannten Reste tragen kann, bedeutet;
A
Sauerstoff oder Schwefel;
X
Stickstoff oder eine Methingruppe = $CR^8$-, worin
$R^8$
einen der Reste $R^3$ bedeutet oder $R^8$ und $R^3$ gemeinsam eine 1,3-Butadien-1,4-diyl- oder eine Aza-1,3-butadien-1,4-diylkette bedeuten, wobei diese Ketten ihrerseits ein bis vier Halogenatome und/oder ein oder zwei der bei $R^1$ genannten Reste tragen können;
Y, Z
Stickstoff oder eine Methingruppe = CH-,
sowie deren landwirtschaftlich brauchbaren Salze.

Weiterhin betrifft die Erfindung Verfahren zur Herstellung der Verbindungen I sowie ihre Verwendung als Herbizide und Wachstumsregulatoren.

In der Literatur (EP-A 223 406, EP-A 249 708, EP-A 287 072 und EP-A 287 079) werden herbizid wirksame Salizylsäurederivate und deren Schwefelanaloge beschrieben.

Der vorliegenden Erfindung lagen Verbindungen als Aufgabe zugrunde, die hinsichtlich der Kulturpflanzenverträglichkeit und Aufwandmenge verbesserte herbizide und wachstumsregulierende Eigenschaften haben.

Demgemäß wurden die eingangs definierten Carbonsäurederivate I gefunden.

Außerdem wurden Verfahren zur Herstellung der Verbindungen, Verfahren zur Bekämpfung unerwünschten Pflanzenwachstums und Verfahren zur Beeinflussung des Pflanzenwuchses mit den Verbindungen I und die entsprechenden Mittel gefunden.

Die Carbonsäurederivate der Formel I sind auf verschiedenen Wegen zugänglich. So erhält man sie beispielsweise besonders vorteilhaft, indem man eine entsprechende aromatische ortho-Hydroxy- bzw. ortho-Mercaptocarbonsäure der Formel II in an sich bekannter Weise in Gegenwart einer Base mit einem Heterocyclus der Formel III umsetzt.

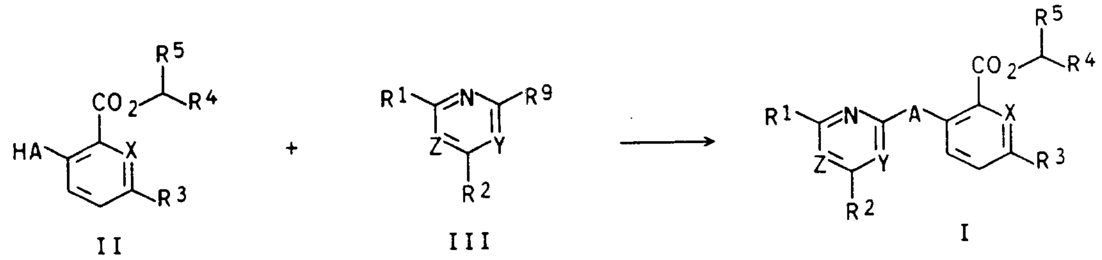

II + III → I

In Formel III bedeutet $R^9$ eine Abgangsgruppe wie Halogen, z.B. Fluor, Chlor, Brom und Jod, oder Aryl- oder Alkylsulfonyl wie Toluolsulfonyl, Methylsulfonyl und Trifluormethylsulfonyl.

Als Basen kommen Alkalimetall- oder Erdalkalimetallhydride, -hydroxide, -carbonate, -hydrogencarbonate und -amide ebenso in Betracht wie Alkyl- oder Arylalkalimetallverbindungen, Alkalimetall- und Erdalkalimetallalkoholateund tertiäre Amine.

Insbesondere finden bei dieser Umsetzung Natrium- oder Calciumhydrid, Natrium- oder Kaliumhydroxid, -carbonat bzw. -hydrogencarbonat, Natriummethylat, Kalium-tert.-butylat und Natriumamid als Basen Verwendung.

Bei Verwendung von anorganischen Basen kann es von Vorteil für die Umsetzungsgeschwindigkeit sein, der Reaktionsmischung einen Phasentransfer-Katalysator wie Kronenether und organische Ammoniumsalze zuzusetzen.

Die für die Umsetzung benötigten Heterocyclen III sind bekannt oder in bekannter Weise erhältlich.

Außerdem kann man die Verbindungen I erhalten, indem man eine Carbonsäure IV zunächst in an sich bekannter Weise in das Halogenid oder eine andere aktivierte Form der Carbonsäure überführt und diese Derivate anschließend mit einem Alkohol der Formel V verestert.

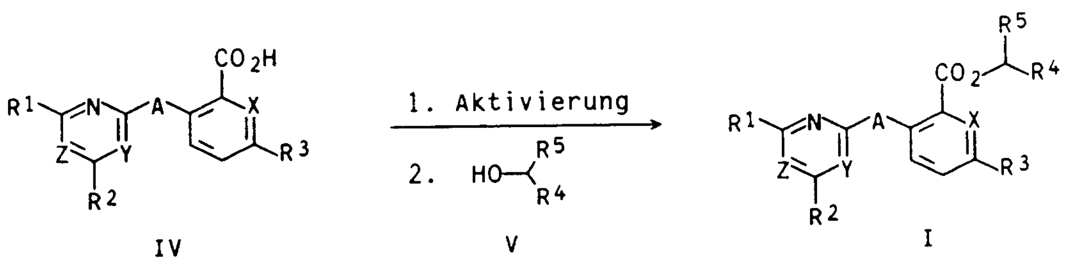


IV V I

Aktivierte Formen der Carbonsäure sind neben Halogeniden wie Chlorid, Bromid und Jodid auch Imidazolide.

Die für die Veresterung benötigten Carbonsäuren IV sind bekannt. Die Alkohole der Formel V lassen sich nach bekannten Methoden herstellen.

Im Hinblick auf die herbizide Wirksamkeit sind Verbindungen I bevorzugt, in denen die Substituenten folgende Bedeutung haben:

$R^1$, $R^2$

Alkyl wie Methyl, Ethyl, Propyl, 1-Methylethyl, Butyl, 1-Methylpropyl, 2-Methylpropyl und 1,1-Dimethylethyl, vorzugsweise Methyl, Ethyl und 1-Methylethyl,

Halogenalkyl wie Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2, 2, 2-Trifluorethyl, 2-Chlor-2,2-difluorethyl, 2,2-Dichlor-2-fluorethyl, 2, 2, 2-Trichlorethyl und Pentafluorethyl, insbesondere Difluormethyl, Trifluormethyl, 2, 2, 2-Trifluorethyl und Pentafluorethyl, vorzugsweise Difluormethyl und Trifluormethyl;

Alkoxy wie insbesondere Methoxy, Ethoxy, Propyloxy, 1-Methylethoxy, Butyloxy, 1-Methylpropyloxy, 2-Methylpropyloxy und 1,1-Dimethylethoxy, vorzugsweise Methoxy und Ethoxy;

Halogenalkoxy wie Difluormethoxy, Trifluormethoxy, Chlordifluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, vorzugsweise Difluormethoxy und Trifluormethoxy und/oder

Alkylthio wie Methylthio, Ethylthio, Propylthio, 1-Methylethylthio, Butylthio, 1-Methylpropylthio, 2-Methylpropylthio und 1,1-Dimethylethylthio, vorzugsweise Methylthio und Ethylthio;

$R^3$

Wasserstoff; Hydroxy; Cyano; Nitro; Amino; Formyl;

Halogen wie Fluor, Chlor, Brom und Jod, vorzugsweise Fluor und Chlor; Alkyl wie im allgemeinen und im besonderen bei $R^1$ genannt;

Alkoxy wie bei $R^1$ genannt, vorzugsweise Methoxyx;

Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl, insbesondere 2-Propenyl, 2-Butenyl, 3-Methyl-2-butenyl und 3-Methyl-2-pentenyl, vorzugsweise 2-Propenyl und 2-Butenyl;

Alkenyloxy wie Prop-2-enyloxy; But-2-enyloxy, But-3-enyloxy, 1-Methylprop-2-enyloxy, 2-Methylprop-2-enyloxy, Pent-2-enyloxy, Pent-3-enyloxy, Pent-4-enyloxy, 1-Methylbut-2-enyloxy, 2-Methylbut-2-enyloxy, 3-Methylbut-2-1-Methylbut-3-enyloxy, 2-Methylbut-3-enyloxy, 3-Methylbut-3-enyloxy, 1,1-Dimethylprop-2-enyloxy, 1,2-Dimethylprop-2-enyloxy, 1-Ethylprop-2-enyloxy, Hex-2-enyloxy, Hex-3-enyloxy, Hex-4-enyloxy, Hex-5-enyloxy, 1-Methylpent-2-enyloxy, 2-Methylpent-2-enyloxy, 3-Methylpent-2-enyloxy, 4-Methylpent-2-enyloxy, 1-Methylpent-3-enyloxy, 2-Methylpent-3-enyloxy, 3-Methylpent-3-enyloxy, 4-Methylpent-3-enyloxy, 1-Methylpent-4-enyloxy, 2-Methylpent-4-enyloxy, 3-Methylpent-4-enyloxy, 4-Methylpent-4-enyloxy, 1,1-Dimethylbut-2-enyloxy, 1,2-Dimethylbut-2-enyloxy, 1,3-Dimethylbut-2-enyloxy, 2, 3-Dimethylbut-2-enyloxy, 1,1-Dimethylbut-3-enyloxy, 1,2-Dimethylbut-3-enyloxy, 1,3-Dimethylbut-3-enyloxy, 2,3-Dimethylbut-3-enyloxy, 2,2-Dimethylbut-3-enyloxy, , 1-Ethylbut-2-enyloxy, 2-Ethylbut-2-enyloxy, 1-Ethylbut-3-enyloxy, 2-Ethylbut-3-enyloxy, 1,1,2-Trimethylprop-2-enyloxy, 1-Ethyl-1-methylprop-2-enyloxy und 1-Ethylen-2-methylprop-2-enyloxy, vorzugsweise 2-Propenyloxy und 2-Butenyloxy;

Alkinyl wie 2-Propinyl, 2-Butinyl, 3-Butinyl, 1-Methyl-2-propinyl, 2-Pentinyl, 3-Pentinyl, 4-Pentinyl, 1-Methyl-2-butinyl, 1-Methyl-3-butinyl, 2-Methyl-3-butinyl, 1,1-Dimethyl-2-propinyl, 1-Ethyl-2-propinyl, 2-Hexinyl, 3-Hexinyl, 4-Hexynyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl, vorzugsweise 2-Propinyl und 2-Butinyl;

Alkinyloxy wie Prop-2-inyloxy, But-2-inyloxy, But-3-inyloxy, 1-Methylprop-2-inyloxy, Pent-2-inyloxy, Pent-3-inyloxy, Pent-4-inyloxy, 1-Methylbut-2-inyloxy, 1-Methylbut-3-inyloxy, 2-Methylbut-3-inyloxy, 1,1-Dimethylprop-2-inyloxy, 1-Ethylprop-2-inyloxy, Hex-2-inyloxy, Hex-3-inyloxy, Hex-4-inyloxy, Hex-5-inyloxy, 1-Methylpent-2-inyloxy, 1-Methylpent-3-inyloxy, 1-Methylpent-4-inyloxy, 2-Methylpent-3-inyloxy, 2-Methylpent-4-inyloxy, 3-Methylpent-4-inyloxy, 4-Methylpent-2-inyloxy, 1,1-Dimethylbut-2-inyloxy, 1,1-Dimethylbut-3-inyloxy, 1,2-Dimethylbut-3-inyloxy, 2,2-Dimethylbut-3-inyloxy, 1-Ethylbut-2-inyloxy, 1-Ethylbut-3-inyloxy, 2-Ethylbut-3-inyloxy und 1-Ethyl-1-methylprop-2-inyloxy, vorzugsweise 2-Propinyloxy und 2-Butinyloxy;

wobei die vorstehend genannten Kohlenstoffreste ein bis fünf Halogenatome wie vorstehend genannt, vorzugsweise Fluor und insbesondere Chlor

und/oder eine

Alkoxygruppe wie im allgemeinen und im besonderen bei $R^1$ genannt;
oder eine
Alkylthiogruppe wie bei $R^1$ genannt, vorzugsweise Methylthio tragen können;
Alkylamino wie
Methylamino, Ethylamino, Propylamino, 1-Methylethylamino, Butylamino, 1-Methylpropylamino, 2-Methylpropylamino und 1,1-Dimethylethylamino;
Dialkylamino wie Di-Methylamino, Di-Ethylamino, Di-Propylamino, Di-1-Methylethylamino, Di-Butylamino, Di-1-Methylpropylamino, Di-2-Methylpropylamino, Di-1,1-Dimethylethylamino, Ethylmethylamino, Propylmethylamino, 1-Methylethylmethylamino, Butylmethylamino, 1-Methylpropylmethylamino, 2-Methylpropylmethylamino, 1,1-Dimethylethylmethylamino, Propylethylamino, 1-Methylethylethylamino, Butylethylamino, 1-Methylpropylethylamino, 2-Methylpropylethylamino, 1,1-Dimethylethylethylamino, 1-Methylethylpropylamino, Butylpropylamino, 1-Methylpropylpropylamino, 2-Methylpropylpropylamino, 1,1-Dimethylethylpropylamino, 1-Methylethylbutylamino, 1-Methylpropylbutylamino, 2-Methylpropylbutylamino und 1,1-Dimethylethylbutylamino;
Phenylamino;
Alkylphenylamino wie N-Methyl-N-phenylamino, N-Ethyl-N-phenylamino, N-Phenyl-N-propylamino, N-(1-Methylethyl)-N-phenylamino, N-Butyl-N-phenylamino, N-(1-Methylpropyl)-N-phenylamino, N-(2-Methylpropyl)-N-phenylamino und N-(1,1-Dimethylethyl)-N-phenylamino;
oder
Alkylcarbonylamino wie Methylcarbonylamino, Ethylcarbonylamino, Propylcarbonylamino, 1-Methylethylcarbonylamino, Butylcarbonylamino, 1-Methylpropylcarbonylamino, 2-Methylpropylcarbonylamino, 1,1-Dimethylethylcarbonylamino, Pentylcarbonylamino, 1-Methylbutylcarbonylamino, 2-Methylbutylcarbonylamino, 3-Methylbutylcarbonylamino, 1,1-Dimethylpropylcarbonylamino, 1,2-Dimethylpropylcarbonylamino, 2,2-Dimethylpropylcarbonylamino, 1-Ethylpropylcarbonylamino, Hexylcarbonylamino, 1-Methylpentylcarbonylamino, 2-Methylpentylcarbonylamino, 3-Methylpentylcarbonylamino, 4-Methylpentylcarbonylamino, 1,1-Dimethylbutylcarbonylamino, 1,2-Dimethylbutylcarbonylamino, 1,3-Dimethylbutylcarbonylamino, 2,2-Dimethylbutylcarbonylamino, 2,3-Dimethylbutylcarbonylamino, 3,3-Dimethylbutylcarbonylamino, 1-Ethylbutylcarbonylamino, 2-Ethylbutylcarbonylamino, 1, 1,2-Trimethylpropylcarbonylamino, 1,2,2-Trimethylpropylcarbonylamino, 1-Ethyl-1-methylpropylcarbonylamino und 1-Ethyl-2-methylpropylcarbonylamino;
$R^4$
Wasserstoff oder
Alkyl wie bei $R^1$ genannt, vorzugsweise Methyl, Ethyl, Propyl und 1-Methylethyl;
$R^5$
5-gliedriges Heteroaryl wie Oxadiazolyl, Thiadiazolyl, Oxazolyl, Isoxazolyl, Thiazolyl und Isothiazolyl, vorzugsweise Oxazolyl, Isoxazolyl, Oxadiazolyl, Thiadiazolyl, Thiazolyl und Isothiazolyl
oder
Isoxazolinyl,
wobei diese cyclischen Reste ein bis vier Halogenatome wie bei $R^3$ genannt, vorzugsweise Fluor und Chlor;
und/oder ein oder zwei der folgenden Reste tragen können:
Alkyl mit 1 bis 6 Kohlenstoffen, vorzugsweise die bei $R^1$ im allgemeinen genannten Gruppen;
Halogenalkyl wie bei $R^1$ genannt, vorzugsweise Trifluormethyl ;
Alkoxy wie bei $R^1$ genannt, vorzugsweise Methoxy und Ethoxy;
Halogenalkoxy wie im allgemeinen und im besonderen bei $R^1$ genannt;
Cycloalkyl wie Cyclopropyl, Cyclobutyl, Cyclopentyl, Cyclohexyl, Cycloheptyl und Cyclooctyl;
Phenyl oder Pyridyl, wobei die aromatischen Reste ihrerseits ein bis fünf Halogenatome wie bei $R^3$ genannt, vorzugsweise Fluor und Chlor
und/oder ein bis drei der im allgemeinen und im besonderen bei $R^1$ genannten Reste tragen können;
oder
eine Gruppe $-CR^6=NOR^7$, worin die Substituenten folgende Bedeutung haben:
$R^6$
Wasserstoff;
Alkyl wie bei $R^1$ genannt, vorzugsweise Methyl, Ethyl, Propyl und 1-Methylethyl,
welches ein bis fünf Halogenatome wie bei $R^3$ genannt, vorzugsweise Fluor und Chlor
und/oder einen Phenylring oder eine der im allgemeinen und im besonderen bei $R^1$ genannten Alkoxy- oder Alkylthiogruppen tragen kann, wobei der Phenylring seinerseits ein bis fünf Halogenatome und/oder ein bis drei der im allgemeinen und im besonderen bei $R^1$ genannten Reste tragen kann;
Cycloalkyl wie vorstehend genannt, vorzugsweise Cyclopropyl, Cyclopentyl und Cyclohexyl ;
welches ein bis drei der bei $R^1$ im allgemeinen und im besonderen genannten Alkylgruppen tragen kann;

Phenyl, welches ein bis fünf Halogenatome wie bei $R^3$ genannt, vorzugsweise Fluor und Chlor, und/oder ein bis drei der im allgemeinen und im besonderen bei $R^1$ genannten Reste tragen kann;

$R^7$

Alkyl mit 1 bis 8 Kohlenstoffatomen, vorzugsweise mit 1 bis 4 Kohlenstoffatomen, wie bei $R^1$ genannt;

Alkenyl wie bei $R^3$ genannt,

wobei diese Reste ein bis fünf Halogenatome wie bei $R^3$ genannt, vorzugsweise Fluor und Chlor und/oder einen der im allgemeinen und im besonderen bei $R^1$ genannten Reste tragen kann;

Cycloalkyl wie vorstehend genannt, vorzugsweise Cyclopropyl, Cyclopentyl und Cyclohexyl;

welches ein bis drei der bei $R^1$ im allgemeinen und im besonderen genannten Alkylgruppen tragen kann;

Phenyl, welches ein bis fünf Halogenatome wie bei $R^3$ genannt, vorzugsweise Fluor und Chlor und/oder ein bis drei der im allgemeinen und im besonderen bei $R^1$ genannten Reste tragen kann;

A

ein Sauerstoff- oder ein Schwefelatom;

X

ein Stickstoffatom oder eine Methingruppe $=CR^8$-, worin

$R^8$

einen der im allgemeinen und im besonderen bei $R^3$ genannten Rest bedeutet oder

$R^8$ und $R^3$ gemeinsam

eine 1,3-Butadien-1,4-diylkette oder eine Aza-1,3-butadien-1,4-diylkette wie 1-Azabuta-1,3-dien-1,4-diyl und 2-Azabuta-1,3-dien-1,4-diyl bedeuten, wobei diese Ketten ihrerseits ein bis vier Halogenatome wie bei $R^3$ genannt, vorzugsweise Fluor, Chlor und Brom und/oder ein oder zwei der im allgemeinen und im besonderen bei $R^1$ genannten Reste tragen können;

Y, Z

Stickstoff oder eine Methingruppe $=CH$-,

sowie deren landwirtschaftlich brauchbaren Salze.

Besonders bevorzugte Carbonsäurederivate der Formel I sind in den folgenden Tabellen A und B wiedergegeben.

Tabelle A

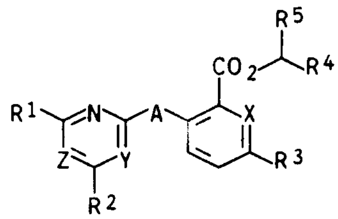

| $R^1$ | $R^2$ | Z | Y | A | X | $R^3$ | $R^4$ | $R^5$ |
|---|---|---|---|---|---|---|---|---|
| $OCH_3$ | $OCH_3$ | CH | N | O | =CF– | H | H | $C(CH_3)=NOCH_3$ |
| $OCH_3$ | $OCH_3$ | CH | N | O | =CF– | H | H | $CH=NOCH_2C_6H_5$ |
| $OCH_3$ | $OCH_3$ | CH | N | O | =CF– | H | H | $CH=NOCH_2CH_3$ |
| $OCH_3$ | $OCH_3$ | CH | N | O | =N– | H | H | $C(CH_3)=NOCH_2CH_3$ |
| $OCH_3$ | $OCH_3$ | CH | N | O | =CCl– | H | H | $CH=NOCH_2CH=CH_2$ |
| $OCH_3$ | $OCH_3$ | CH | N | O | =N– | H | H | $CH=NOCH_2CH_3$ |
| $OCH_3$ | $OCH_3$ | CH | N | O | =CCl– | H | H | $C(CH_3)=NOCH_2CH_3$ |
| $OCH_3$ | $OCH_3$ | CH | N | O | =CCl– | H | H | $CH=NOCH_2C_6H_5$ |
| $OCH_3$ | $OCH_3$ | CH | N | O | =CCl– | H | H | $C(CH_3)=NOCH_2C_6H_5$ |
| $OCH_3$ | $OCH_3$ | CH | N | O | =CCl– | H | H | $CH=NOCH_3$ |
| $OCH_3$ | $OCH_3$ | CH | N | O | =CCl– | H | H | $CH=NOCH_2CH=CHCl$ |
| $OCH_3$ | $OCH_3$ | CH | N | O | =N– | H | H | $CH=NOCH_2C_6H_5$ |
| $OCH_3$ | $OCH_3$ | CH | N | O | =CCl– | H | H | $C(CH_3)=NOCH_2CH=CH_2$ |
| $OCH_3$ | $OCH_3$ | CH | N | O | =CCl– | H | H | 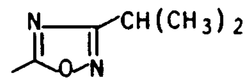 |
| $OCH_3$ | $OCH_3$ | CH | N | O | =CCl– | H | H | 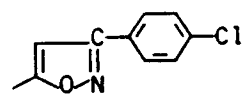 |
| $OCH_3$ | $OCH_3$ | CH | N | O | =CCl– | H | H | $CH=NOCH_2CH_3$ |
| $OCH_3$ | $OCH_3$ | CH | N | O | =CCl– | H | H | 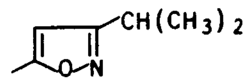 |
| $OCH_3$ | $OCH_3$ | CH | N | O | $=C(CH_3)-$ | H | H | 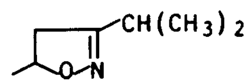 |
| $OCH_3$ | $OCH_3$ | CH | N | O | =CCl– | H | H | 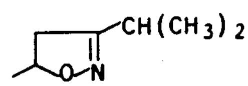 |
| $OCH_3$ | $OCH_3$ | CH | N | O | $=C(OCH_3)-$ | H | H | $CH=NOCH_2CH_3$ |

Tabelle A (Fortsetzung)

| $R^1$ | $R^2$ | Z | Y | A | X | $R^3$ | $R^4$ | $R^5$ |
|---|---|---|---|---|---|---|---|---|
| $OCH_3$ | $OCH_3$ | CH | N | O | =CCl− | H | $CH_3$ | $CH=NOCH_2CH_3$ |
| $OCH_3$ | $CF_3$ | CH | N | O | =CF− | H | H | $CH=NOCH_2CH=CHCl$ |
| $OCH_3$ | $OCH_3$ | CH | N | O | $=C(OCH_2CH=CH_2)-$ | H | H | $CH=NOCH_3$ |
| $OCH_3$ | $OCH_3$ | CH | N | S | =CCl− | H | H | $CH=NOCH_2CH_3$ |
| $OCH_3$ | $OCH_3$ | CH | N | S | =CCl− | H | H | $C(CH_3)=NOCH_2CH_3$ |
| $OCH_3$ | $OCH_3$ | CH | N | S | =CCl− | H | H | $CH=NOCH_2-$ 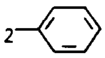 |
| $OCH_3$ | $OCH_3$ | CH | N | S | =CCl− | H | H | $CH=NOCH_2CH=CH_2$ |
| $OCH_3$ | $OCH_3$ | CH | N | S | =CCl− | H | H | $CH=NOCH_2CH=CHCl$ |
| $OCH_3$ | $OCH_3$ | CH | N | S | =CCl− | H | H | $C(CH_3)=NOCH_2-$ 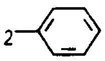 |
| $OCH_3$ | $OCH_3$ | N | N | S | =CCl− | H | H | $CH=NOCH_2CH_3$ |
| $OCH_3$ | $OCH_3$ | N | N | O | =CCl− | H | H | $CH=NOCH_2CH_3$ |
| $OCH_3$ | $OCH_3$ | N | N | O | =CCl− | H | H | $C(CH_3)=NOCH_2CH_3$ |
| $OCH_3$ | $OCH_3$ | N | N | O | =CF− | H | H | $CH=NOCH_2CH_3$ |
| $OCH_3$ | $OCH_3$ | N | N | O | =CCl− | H | H | $CH=NOCH_2-$ 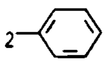 |
| $OCH_3$ | $OCH_3$ | N | N | O | =CCl− | H | H | $CH=NOCH_2CH=CH_2$ |
| $OCH_3$ | $SCH_3$ | N | N | O | =CCl− | H | H | $CH=NOCH_3$ |
| $OCH_3$ | $OCH_3$ | CH | N | O | $=C(CF_3)-$ | H | H | $C(CH_3)=NOCH_2-$ 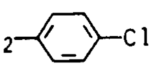 |
| $OCH_3$ | $OCH_3$ | CH | N | O | $=C(OCH_2SCH_3)-$ | H | H | $CH=NOCH_2CH=CH_2$ |
| $OCH_3$ | $OCH_3$ | CH | N | O | =CCl− | H | H | $CH=NOCH_2CH=CHCH_3$ |
| $OCH_3$ | $OCH_3$ | CH | N | O | =CCl− | H | H | 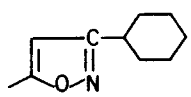 |
| $OCH_3$ | $OCH_3$ | CH | N | O | =CCl− | H | H | 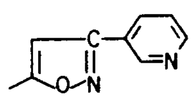 |

Tabelle B

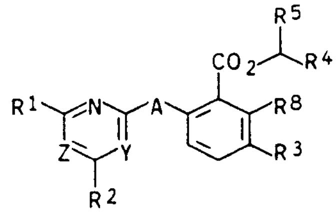

| $R^1$ | $R^2$ | Z | Y | A | $R^8$ | $R^3$ | $R^4$ | $R^5$ |
|---|---|---|---|---|---|---|---|---|
| $OCH_3$ | $OCH_3$ | CH | N | O | CH=CH–CH=CH | | H | 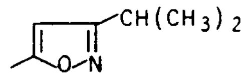 |
| $OCH_3$ | $OCH_3$ | CH | N | O | CH=CH–CH=CH | | H | $CH{=}NOCH_2CH{=}CHCl$ |
| $OCH_3$ | $OCH_3$ | CH | N | O | CH=CH–CH=CH | | H | $C(CH_3){=}NOCH_3$ |
| $OCH_3$ | $OCH_3$ | CH | N | O | CH=CH–CH=CH | | H | $CH{=}NOCH_2CH_3$ |
| $OCH_3$ | $OCH_3$ | CH | N | O | CH=CH–CH=CH | | H | $CH{=}NOCH_2$– 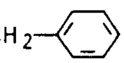 |
| $OCH_3$ | $OCH_3$ | CH | N | O | CH=CH–CH=CH | | H | $C(CH_3){=}NOCH_2$– 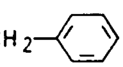 |
| $OCH_3$ | $OCH_3$ | CH | N | O | CH=CH–CH=CH | | H | $CH{=}NOCH_2CH{=}CH_2$ |
| $OCH_3$ | $OCH_3$ | CH | N | O | CH=CH–CH=CH | | H | $CH{=}NOCH_2$– 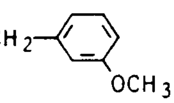 |
| $OCH_3$ | $OCH_3$ | N | N | O | CH=CH–CH=CH | | H | $CH{=}NOCH_2CH_3$ |
| $OCH_3$ | $OCH_3$ | CH | N | O | CH=CH–CBr=CH | | H | $CH{=}NOCH_2CH_3$ |
| $OCH_3$ | $OCH_3$ | CH | N | O | CF=CH–CH=CH | | H | $CH{=}NOCH_2CH_3$ |
| $OCH_3$ | $OCH_3$ | CH | N | O | CH=CH–CH=CH | | H | $C(CH_3){=}NOCH_2CH{=}CH_2$ |
| $OCH_3$ | $OCH_3$ | CH | N | O | $CH{=}CH{-}C(CH_3){=}CH$ | | H | $CH{=}NOCH_3$ |

Als Salze der Verbindungen der Formel I kommen landwirtschaftlich brauchbare Salze, beispielsweise Alkalimetallsalze, wie das Kalium- oder Natriumsalz, Erdalkalimetallsalze, wie das Calcium-, Magnesium- oder Bariumsalz, Mangan-, Kupfer-, Zink- oder Eisensalze sowie Ammonium, Phosphonium-, Sulfonium- oder Sulfoxoniumsalze, beispielsweise Ammoniumsalze, Tetraalkylammoniumsalze, Benzyltrialkylammoniumsalze, Trialkylsulfoniumsalze oder Trialkylsulfoxoniumsalze in Betracht.

Die erfindungsgemäßen herbiziden und wachstumsregulierenden Verbindungen I bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0, 1 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können beispielsweise wie folgt formuliert werden:

I. Man vermischt 90 Gewichtsteile der Verbindung Nr. 1.016 mit 10 Gewichtsteilen N-Methyl-$\alpha$-pyrrolidon und erhält eine Lösung, die zur Anwendung in Form kleinster Tropfen geeignet ist.

II. 20 Gewichtsteile der Verbindung Nr. 1.003 werden in einer Mischung gelöst, die aus 80 Gewichtsteilen Xylol, 10 Gewichtsteilen des Anlagerungsproduktes von 8 bis 10 Mol Ethylenoxid an 1 Mol ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 2.003 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 2.006 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0, 02 Gew.% des Wirkstoffs enthält.

V. 20 Gewichtsteile des Wirkstoffs Nr. 1.014 werden mit 3 Gewichtsteilen des Natriumsalzes der Diisobutylnaphthalin-$\alpha$-sulfonsäure` 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 1.016 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 1.015 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 2.004 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der herbiziden und wachstumsregulierenden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff bei Anwendung als Herbizide betragen je nach Bekämpfungsziel, Jahreszeit, Zielpflanzen und Wachstumsstadium 0,001 bis 3, vorzugsweise 0,01 bis 1 kg/ha aktive Substanz (a.S.).

Die Verbindungen der Formel I können praktisch alle Entwicklungsstadien einer Pflanze verschiedenartig beeinflussen und werden deshalb als Wachstumsregulatoren eingesetzt. Die Wirkungsvielfalt der Pflanzenwachstumsregulatoren hängt ab vor allem

a) von der Pflanzenart und -sorte,

b) von dem Zeitpunkt der Applikation, bezogen auf das Entwicklungsstadium der Pflanze und von der Jahreszeit,

c) von dem Applikationsort und -verfahren z.B. (Samenbeize, Bodenbehandlung, Blattapplikation oder Stamminjektion bei Bäumen)

d) von klimatischen Faktoren, z.B. Temperatur, Niederschlagsmenge, außerdem auch Tageslänge und Lichtintensität

e) von der Bodenbeschaffenheit (einschließlich Düngung),

f) von der Formulierung bzw. Anwendungsform des Wirkstoffs und schließlich

g) von den angewendeten Konzentrationen der aktiven Substanz.

Aus der Reihe der verschiedenartigen Anwendungsmöglichkeiten der erfindungsgemäßen Pflanzenwachstumsregulatoren der Formel I im Pflanzenanbau, in der Landwirtschaft und im Gartenbau, werden eingige nachstehend erwähnt.

A. Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf; außerdem ist eine dunklere Blattfärbung zu beobachten.

Als vorteilhaft für die Praxis erweist eine verminderte Intensität des Wachstums von Gräsern an Straßenrändern, Hecken, Kanalböschungen und auf Rasenflächen wie Park-, Sport- und Obstanlagen, Zierrasen und Flugplätzen, so daß der Arbeits- und kostenaufwendige Rasenschnitt reduziert werden kann.

Von wirtschaftlichem Interesse ist auch die Erhöhung der Standfestigkeit von lageranfälligen Kulturen wie Getreide, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.

Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.

Bei Obst- und anderen Bäumen lassen sich mit den Wachstumsregulatoren Schnittkosten einsparen. Außerdem kann die Alternanz von Obstbäumen durch Wachstumsregulatoren gebrochen werden.

Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit Wachstumsregulatoren läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generativen Phase neigen. Auch bei anderen Kulturen z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und -

wegen der relativ geringen Blatt- bzw. Pflanzenmasse - dem Befall mit verschiedenen Krankheiten (z.B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag bezogen auf die Bodenfläche erzielt werden kann.

B. Mit den Wachstumsregulatoren lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.

Dabei können die Verbindungen der Formel I Ertragssteigerungen durch Eingriffe in den pflanzlichen Stoffwechsel bzw. durch Förderung oder Hemmung des vegetativen und/oder des generativen Wachstums verursachen.

C. Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- bzw. Blatt- und Sproßteil der Pflanze ist auch für ein gut kontrollierbares Entblättern von Nutzpflanzen wie beispielsweise Baumwolle wesentlich.

D. Mit Wachstumsregulatoren kann weiterhin der Wasserverbrauch von Pflanzen reduziert werden. Dies ist besonders wichtig für landwirtschaftliche Nutzflächen, die unter einem hohen Kostenaufwand künstlich bewässert werden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es zu einer besseren Ausnutzung des vorhandenen Wassers, weil u.a.

- die Öffnungsweite der Stomata reduziert wird
- eine dickere Epidermis und Cuticula ausgebildet werden
- die Durchwurzelung des Bodens verbessert wird und
- das Mikroklima im Pflanzenbestand durch einen kompakteren Wuchs günstig beeinflußt wird.

Die erfindungsgemäß zu verwendenden Wachstumsregulatoren der Formel I können den Kulturpflanzen sowohl vom Samen her (als Saatgutbeizmittel) als auch über den Boden, d.h. durch die Wurzel sowie - besonders bevorzugt - durch Spritzung über das Blatt zugeführt werden.

Infolge der hohen Pflanzenverträglichkeit kann die Aufwandmenge stark variiert werden.

In Anbetracht der Vielseitigkeit der Applikationsmethoden können die erfindungsgemäßen Verbindungen bzw. sie enthaltende Mittel in einer großen Zahl von Kulturpflanzen zur Beseitigung unerwünschter Pflanzen eingesetzt werden.

Zur Verbreiterung des Wirkungsspektrums und zur Erzielung synergistischer Effekte können die erfindungsgemäßen Verbindungen I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Aryloxy-, Heteroaryloxyphenoxypropionsäuren sowie deren Salze, Ester und Amide und andere in Betracht.

Außerdem kann es von Nutzen sein, die Verbindungen I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs-und Spurenelementmängeln eingesetzt werden. Es können auch nichtphytotoxische Öle und ölkonzentrate zugesetzt werden.

Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den nachstehenden Tabellen mit physikalischen Angaben aufgeführt.

Beispiel 1

Herstellung von 2-(4,6-Dimethoxypyrimidin-2-yl)oxynaphthalin-1-carbonsäure-(3-isopropylisoxazol-5-yl)-methylester

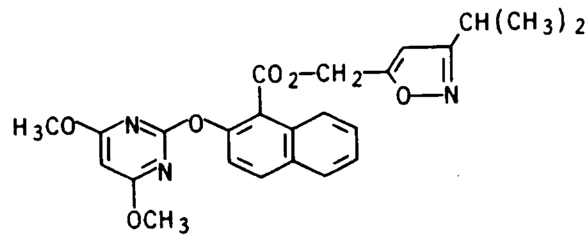

a) 2-(4,6-Dimethoxypyrimidin-2-yl)oxynaphthalin-1-carbonsäure

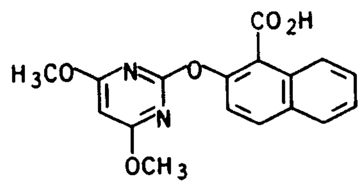

Eine Mischung aus 14,7 g 2-Hydroxynaphthalin-1-carbonsäure und 640 ml Methanol wurde bei 25 °C mit 5,1 g Kaliumhydroxid versetzt. Nach ca. 10 min. wurde das Lösungsmittel bei vermindertem Druck entfernt. Das so erhaltene Kaliumsalz wurde getrocknet und anschließend in 380 ml Dimethylsulfoxid gelöst. Zu dieser Lösung gab man bei 25 °C portionsweise 2,52 g (80 %ige Suspension in Leinöl) Natriumhydrid. Nach weiteren 30 min. wurde die klare Lösung mit 17,4 g 4,6-Dimethoxy-2-methylsulfonylpyrimidin versetzt. Die Umsetzung war nach ca. 12 Std. beendet. Zur Aufarbeitung wurde das Reaktionsgemisch mit Wasser versetzt und mittels Essigsäureethylester-Extraktion von Verunreinigungen befreit. Durch Ansäuern der wäßrigen Phase erhielt man das gewünschte Produkt als Feststoff. Ausbeute: 22 g

b) 2-(4,6-Dimethoxypyrimidin-2-yl)oxynaphthalin-1-carbonsäure-(3-iso-propylisoxazol-5-yl)methylester

Eine Mischung aus 4,9 g der Naphthalincarbonsäure aus a) und 100 ml Dimethylsulfoxid wurde bei 25 °C nacheinander zunächst mit 1,7 g Kalium-tert.-butylat und dann mit 3,1 g (3-Isopropylisoxazol-5-yl)-methylbromid versetzt. Nach ca. 12 Std. bei 25 °C war die Umsetzung beendet. Zur Aufarbeitung wurde die Reaktionsmischung mit Wasser verdünnt, angesäuert und mit Essigsäureethylester extrahiert. Aus der organischen Phase erhielt man das gewünschte Produkt als Öl.

Ausbeute: 3,6 g [nach Chromatographie an Kieselgel]; $^1$H-NMR (250 mHz) ausgewählte Signale: 1,25 (d) ; 3,02 (m) ; 3,75 (s); 5,35 (s) ; 5,75 (s) ; 6,10 (s)

Wirkstoffbeispiel 2.001

Beispiel 2

Herstellung von 2-(4,6-Dimethoxypyrimidin-2-yl)oxynaphthalin-1-carbonsäure-[2-(3-Chlor-2-propen-1-yl-oxyimino)]ethylester (E-Isomer)

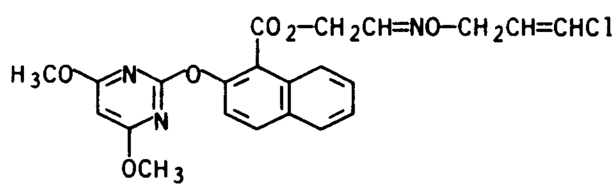

Eine Mischung aus 3,1 g der Naphthalincarbonsäure aus 1a) und 50 ml Dimethylsulfoxid wurde bei 25 °C nacheinander zunächst mit 1,1 g Kalium-tert.-butylat und dann mit 1,7 g 2-(3-Chlor-2-propen-1-yloxyimino)-ethylchlorid (E-Isomer) versetzt. Nach ca. 12 Std. war die Umsetzung beendet. Zur Aufarbeitung wurde die Reaktionsmischung mit Wasser verdünnt, angesäuert und mit Essigsäureethylester extrahiert. Aus der organischen Phase erhielt man das gewünschte Produkt als Feststoff.
Ausbeute: 2,2 g [nach Chromatographie an Kieselgel]; Fp.: 77-83 °C.
Wirkstoffbeispiel 2.002

Tabelle 1

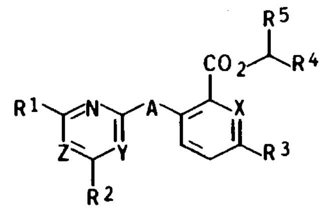

| Nr. | $R^1$ | $R^2$ | Z | Y | A | X | $R^3$ | $R^4$ | $R^5$ | phys. Daten [Fp(°C), NMR* (δ in ppm)] |
|---|---|---|---|---|---|---|---|---|---|---|
| 1.001 | $OCH_3$ | $OCH_3$ | CH | N | O | =CF– | H | H | $C(CH_3)=NOCH_3$ | 71- 74 |
| 1.002 | $OCH_3$ | $OCH_3$ | CH | N | O | =CF– | H | H | $CH=NOCH_2C_6H_5$ | 3,82(s), 4,73(d), 4,55(d), 5,78(s) |
| 1.003 | $OCH_3$ | $OCH_3$ | CH | N | O | =CF– | H | H | $CH=NOCH_2CH_3$ | 1,23(t), 3,84(s), 4,70(d), 4,92(d), 5,78(s) |
| 1.004 | $OCH_3$ | $OCH_3$ | CH | N | O | =N– | H | H | $C(CH_3)=NOCH_2CH_3$ | 74- 77 |
| 1.005 | $OCH_3$ | $OCH_3$ | CH | N | O | =CCl– | H | H | $CH=NOCH_2CH=CH_2$ | 3,93(s), 4,80(d), 5,05(d), 5,80(s), 6,00(m) |
| 1.006 | $OCH_3$ | $OCH_3$ | CH | N | O | =N– | H | H | $CH=NOCH_2CH_3$ | 1,25(t), 3,80(s), 4,78(d), 5,02(d), 5,80(s) |
| 1.007 | $OCH_3$ | $OCH_3$ | CH | N | O | =CCl– | H | H | $C(CH_3)=NOCH_2CH_3$ | 1,23(t), 1,87(s), 3,82(s), 4,10(q), 5,80(s) |
| 1.008 | $OCH_3$ | $OCH_3$ | CH | N | O | =CCl– | H | H | $CH=NOCH_2C_6H_5$ | 3,80(s), 4,80(d), 5,07(s), 5,78(s) |
| 1.009 | $OCH_3$ | $OCH_3$ | CH | N | O | =CCl– | H | H | $C(CH_3)=NOCH_2C_6H_5$ | 1,90(s), 3,80(s), 4,73(s), 5,77(s) |
| 1.010 | $OCH_3$ | $OCH_3$ | CH | N | O | =CCl– | H | H | $CH=NOCH_3$ | 87- 89 |
| 1.011 | $OCH_3$ | $OCH_3$ | CH | N | O | =CCl– | H | H | $CH=NOCH_2CH=CHCl$ | 3,85(s), 4,80(d), 5,00(d), 5,78(s), 6,10(m) |
| 1.012 | $OCH_3$ | $OCH_3$ | CH | N | O | =N– | H | H | $CH=NOCH_2C_6H_5$ | 3,80(s), 4,80(d), 5,05(d), 5,78(d) |
| 1.013 | $OCH_3$ | $OCH_3$ | CH | N | O | =CCl– | H | H | $C(CH_3)=NOCH_2CH=CH_2$ | 1,90(s), 3,82(s), 4,57(d), 4,75(s), 5,78(d) |
| 1.014 | $OCH_3$ | $OCH_3$ | CH | N | O | =CCl– | H | H | 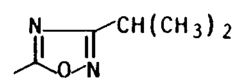 | 1,23(d), 3,78(s), 5,53(s), 6,00(s) |

Tabelle 1 (Fortsetzung)

| Nr. | $R^1$ | $R^2$ | Z | Y | A | X | $R^3$ | $R^4$ | $R^5$ | phys. Daten [Fp(°C), NMR* (δ in ppm)] |
|---|---|---|---|---|---|---|---|---|---|---|
| 1.015 | $OCH_3$ | $OCH_3$ | CH | N | O | =CCl– | H | H | 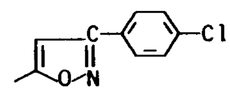 | 127-128 |
| 1.016 | $OCH_3$ | $OCH_3$ | CH | N | O | =CCl– | H | H | $CH{=}NOCH_2CH_3$ | 1,15(t), 3,85(s), 4,80(d), 5,05(d), 5,80(s) |
| 1.017 | $OCH_3$ | $OCH_3$ | CH | N | O | =CCl– | H | H | 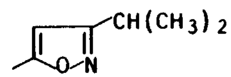 | 1,27(d), 3,85(s), 5,27(s), 5,75(s), 6,13(s), 7,18(d) |
| 1.018 | $OCH_3$ | $OCH_3$ | CH | N | O | N | H | H | 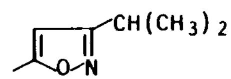 | 1,25(d), 3,80(s), 5,33(s), 5,80(s), 6,20(s), 8,70(d) |

* ausgewählte Signale

Tabelle 2

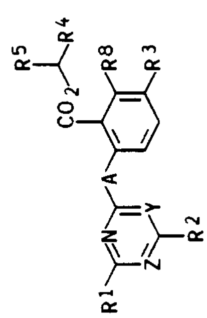

| Nr. | $R^1$ | $R^2$ | Z | Y | A | $R^8$ | $R^3$ | $R^4$ | $R^5$ | phys. Daten [(Fp(°C), NMR (δ in ppm)] |
|---|---|---|---|---|---|---|---|---|---|---|
| 2.0[illegible] | $OCH_3$ | $OCH_3$ | CH | N | O | CH=CH–CH=CH | | H | 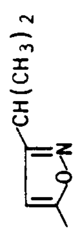 | 1,25(d), 3,02(m), 3,75(s), 5,35(s), 5,75(s), 6,10(s) |
| 2.002 | $OCH_3$ | $OCH_3$ | CH | N | O | CH=CH–CH=CH | | H | $CH=NOCH_2CH=CHCl$ | 77- 83 |
| 2.003 | $OCH_3$ | $OCH_3$ | CH | N | O | CH=CH–CH=CH | | H | $C(CH_3)=NOCH_3$ | 96- 98 |
| 2.004 | $OCH_3$ | $OCH_3$ | CH | N | O | CH=CH–CH=CH | | H | $CH=NOCH_2CH_3$ | 3,82(s), 4,87(d), 5,10(d), 5,80(s) |

Anwendungsbeispiele

Die herbizide Wirkung der Carbonsäuren der Formel I ließ sich durch Gewächshausversuche zeigen: Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde. Die Aufwandmenge für die Vorauflaufanwendung betrug 0,06 kg/ha a.S.

Zum Zwecke der Nachauflaufbehandlung wurden die Testpflanzen je nach Wuchsform erst bei einer Wuchshöhe von 3 bis 15 cm mit den in Wasser suspendierten oder emulgierten Wirkstoffen behandelt. Die Aufwandmenge für die Nachauflaufbehandlung betrug 0,06 kg/ha a.S.

Die Pflanzen wurden artenspezifisch bei Temperaturen von 10-25 °C bzw. 20-35 °C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name |
|---|---|
| Abutilon theophrasti | Chinesischer Hanf |
| Amaranthus retroflexus | Zurückgekrümmter Fuchsschwanz |
| Cassia tora | - |
| Galium aparine | Klettenlabkraut |
| Malva neglecta | Wegmalve |
| Solanum nigrum | Schwarzer Nachtschatten |

Mit 0,06 kg/ha a.S. im Vorauflauf- bzw. Nachauflaufverfahren eingesetzt lassen sich mit den Beispiel-Verbindungen 1.014, 1.015 und 1.016 breitblättrige unerwünschte Pflanzen sehr gut bekämpfen.

## Patentansprüche

**1.** Carbonsäurederivate der allgemeinen Formel I

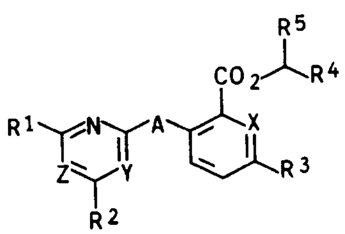

I,

in der die Substituenten folgende Bedeutung haben:

$R^1$, $R^2$

$C_1$-$C_4$-Alkyl, $C_1$-$C_4$-Halogenalkyl, $C_1$-$C_4$-Alkoxy, $C_1$-$C_4$-Halogenalkoxy oder $C_1$-$C_4$-Alkylthio;

$R^3$

Wasserstoff; Hydroxy; Cyano; Nitro; Amino; Formyl;

Halogen;

eine $C_1$-$C_4$-Alkylgruppe, eine $C_1$-$C_4$-Alkoxygruppe, eine $C_3$-$C_6$-Alkenylgruppe, eine $C_3$-$C_6$-Alkenyloxygruppe, eine $C_3$-$C_6$-Alkinylgruppe oder eine $C_3$-$C_6$-Alkinyloxygruppe, wobei diese Gruppen ein bis fünf Halogenatome und/oder eine $C_1$-$C_4$-Alkoxy- oder $C_1$-$C_4$-Alkylthiogruppe tragen können;

eine $C_1$-$C_4$-Alkylaminogruppe; eine Di-$C_1$-$C_4$-Alkylaminogruppe; eine Phenylaminogruppe; eine N-Phenyl-N-$C_1$-$C_4$-alkylaminogruppe oder eine $C_1$-$C_6$-Alkylcarbonylaminogruppe;

$R^4$

Wasserstoff oder $C_1$-$C_4$-Alkyl;

$R^5$

einen fünfgliedrigen Heteroarylrest, der ein oder zwei Stickstoffatome und ein Sauerstoff- oder Schwefelatom enthält, oder einen Isoxazolinylrest, wobei diese Ringsysteme ein bis vier Halogenatome

und/oder ein oder zwei der folgenden Reste tragen können: $C_1$-$C_6$-Alkyl, $C_1$-$C_2$-Halogenalkyl, $C_1$-$C_4$-Alkoxy, $C_1$-$C_2$-Halogenalkoxy, $C_3$-$C_8$-Cycloalkyl, Phenyl oder Pyridyl, wobei die aromatischen Reste ihrerseits ein bis fünf Halogenatome und/oder ein bis drei der bei $R^1$ genannten Reste tragen können; oder eine Gruppe -$CR^6$ = $NOR^7$, worin

$R^6$

Wasserstoff;

$C_1$-$C_4$-Alkyl bedeutet, welches ein bis fünf Halogenatome und/oder einen der folgenden Reste tragen kann: $C_1$-$C_4$-Alkoxy, $C_1$-$C_4$-Alkylthio oder Phenyl, wobei der Phenylrest seinerseits ein bis fünf Halogenatome und/oder ein bis drei der bei $R^1$ genannten Reste tragen kann oder für $C_3$-$C_8$-Cycloalkyl steht, welches ein bis drei $C_1$-$C_4$-Alkylgruppen tragen kann;

Phenyl, welches ein bis fünf Halogenatome und/oder ein bis drei der bei $R^1$ genannten Reste tragen kann, bedeutet und

$R^7$

$C_1$-$C_8$-Alkyl oder $C_3$-$C_6$-Alkenyl, welches ein bis fünf Halogenatome und/oder einen der folgenden Reste tragen kann: $C_1$-$C_4$-Alkoxy, $C_1$-$C_4$-Alkylthio oder Phenyl, wobei der Phenylrest seinerseits ein bis fünf Halogenatome und/oder ein bis drei der bei $R^1$ genannten Reste tragen kann;

$C_3$-$C_8$-Cycloalkyl, welches ein bis drei $C_1$-$C_4$-Alkylgruppen tragen kann; Phenyl, welches ein bis fünf Halogenatome und/oder ein bis drei der bei $R^1$ genannten Reste tragen kann, bedeutet;

A

Sauerstoff oder Schwefel;

X

Stickstoff oder eine Methingruppe = $CR^8$-, worin

$R^8$

einen der Reste $R^3$ bedeutet oder $R^8$ und $R^3$ gemeinsam eine 1,3-Butadien-1,4-diyl- oder eine Aza-1,3-butadien-1,4-diylkette bedeuten, wobei diese Ketten ihrerseits ein bis vier Halogenatome und/oder ein oder zwei der bei $R^1$ genannten Reste tragen können;

Y, Z

Stickstoff oder eine Methingruppe = CH-,

sowie deren landwirtschaftlich brauchbaren Salze.

**2.** Verfahren zur Herstellung der Verbindungen I gemäß Anspruch 1, dadurch gekennzeichnet, daß man ein aromatisches Carbonsäurederivat der Formel II

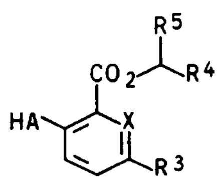

II

in an sich bekannter Weise in Gegenwart einer Base mit einem Heteroaromat der Formel III,

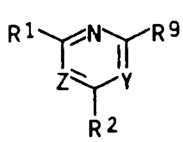

III

worin $R^9$ für eine Abgangsgruppe steht, umsetzt.

**3.** Verfahren zur Herstellung der Verbindungen I gemäß Anspruch 1, dadurch gekennzeichnet, daß man eine aromatische Carbonsäure der Formel IV

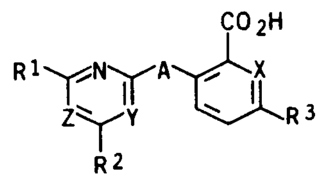

IV

zunächst in an sich bekannter Weise in das Halogenid oder eine andere aktivierte Form der Carbonsäure überführt und diese Derivate anschließend mit einem substituierten Alkohol der Formel V

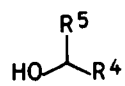

V

verestert.

4. Herbizide Mittel, enthaltend eine Verbindung der Formel I gemäß Anspruch 1 sowie inerte Zusatzstoffe.

5. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Derivates I gemäß Anspruch 1 behandelt.

6. Verwendung von Verbindungen der Formel I gemäß Anspruch 1 als Herbizide.

7. Mittel zur Regulierung des Pflanzenwachstums, enthaltend eine Verbindung I gemäß Anspruch 1 sowie inerte Zusatzstoffe.

8. Verfahren zur Regulierung des Pflanzenwachstums, dadurch gekennzeichnet, daß man eine regulativ wirksame Menge einer Verbindung der allgemeinen Formel I gemäß Anspruch 1 auf die Samen, die Pflanzen und/oder deren Lebensraum einwirken läßt.

9. Verwendung von Verbindungen der Formel I gemäß Anspruch 1 zur Regulierung des Pflanzenwachstums.

## Claims

1. A carboxylic acid derivative of the formula I

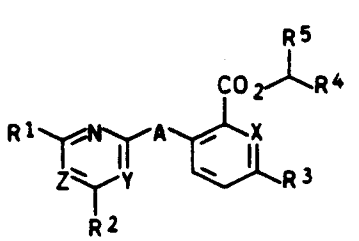

I

where
$R^1$ and $R^2$ are each $C_1$-$C_4$-alkyl, $C_1$-$C_4$-haloalkyl, $C_1$-$C_4$-alkoxy, $C_1$-$C_4$-haloalkoxy or $C_1$-$C_4$-alkylthio;
$R^3$ is hydrogen, hydroxyl, cyano, nitro, amino, formyl, halogen;
$C_1$-$C_4$-alkyl, $C_1$-$C_4$-alkoxy, $C_3$-$C_6$-alkenyl, $C_3$-$C_6$-alkenyloxy, $C_3$-$C_6$-alkynyl or $C_3$-$C_6$-alkynyloxy, where these groups may carry from one to five halogen atoms and/or a $C_1$-$C_4$-alkoxy or $C_1$-$C_4$-alkylthio group;
$C_1$-$C_4$-alkylamino, di-$C_1$-$C_4$-alkylamino, phenylamino, N-phenyl-N-$C_1$-$C_4$-alkylamino or $C_1$-$C_6$-alkylcarbonylamino;

$R^4$ is hydrogen or $C_1$-$C_4$-alkyl;
$R^5$ is a five-membered hetaryl radical which contains one or two nitrogen atoms and an oxygen or sulfur atom, or is an isoxazolinyl radical, where these ring systems may carry from one to four halogen atoms and/or one or two of the following radicals: $C_1$-$C_6$-alkyl $C_1$- or $C_2$-haloalkyl, $C_1$-$C_4$-alkoxy, $C_1$- or $C_2$-haloalkoxy, $C_3$-$C_8$-cycloalkyl, phenyl or pyridyl, where the aromatic radicals in turn may carry from one to five halogen atoms and/or from one to three of the radicals stated for $R^1$;
or a group -$CR^6$ = $NOR^7$, where
$R^6$ is hydrogen;
$C_1$-$C_4$-alkyl which may carry from one to five halogen atoms and/or one of the following radicals: $C_1$-$C_4$-alkoxy, $C_1$-$C_4$-alkylthio or phenyl, where the phenyl radical in turn may carry from one to five halogen atoms and/or from one to three of the radicals stated for $R^1$, or $R^6$ is $C_3$-$C_8$-cycloalkyl which may carry from one to three $C_1$-$C_4$-alkyl groups, or $R^6$ is phenyl which may carry from one to five halogen atoms and/or from one to three of the radicals stated for $R^1$, and
$R^7$ is $C_1$-$C_8$-alkyl or $C_3$-$C_6$-alkenyl which may carry from one to five halogen atoms and/or one of the following radicals: $C_1$-$C_4$-alkoxy, $C_1$-$C_4$-alkylthio or phenyl, where the phenyl radical in turn may carry from one to five halogen atoms and/or from one to three of the radicals stated for $R^1$;
$C_3$-$C_8$-cycloalkyl which may carry from one to three $C_1$-$C_4$-alkyl groups;
phenyl which may carry from one to five halogen atoms and/or from one to three of the radicals stated for $R^1$;
A is oxygen or sulfur;
X is nitrogen or a methine group = $CR^8$-, where
$R^8$ is one of the radicals $R^3$, or $R^8$ and $R^3$ together form a 1,3-butadiene-1,4-diyl or aza-1,3-butadiene-1,4-diyl chain, where these chains in turn may carry from one to four halogen atoms and/or one or two of the radicals stated for $R^1$;
Y and Z are each nitrogen or a methine group = CH-,
and its salts which can be used in agriculture.

2. A process for the preparation of a compound I as claimed in claim 1, wherein an aromatic carboxylic acid derivative of the formula II

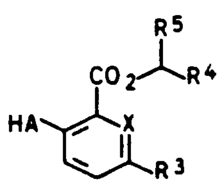

II

is reacted with a heteroaromatic of the formula III

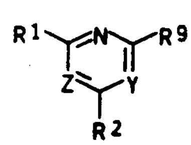

III

where $R^9$ is a leaving group, in a conventional manner in the presence of a base.

3. A process for the preparation of a compound I as claimed in claim 1, wherein an aromatic carboxylic acid of the formula IV

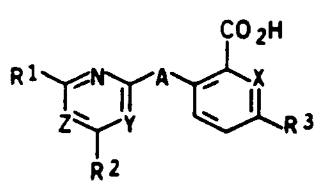

IV

is first converted in a conventional manner into the halide or another activated form of the carboxylic acid, and this derivative is then esterified with a substituted alcohol of the formula V

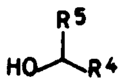

V

**4.** A herbicide containing a compound of the formula I as claimed in claim 1 and inert additives.

**5.** A method for controlling undesirable plant growth, wherein the undesirable plants and/or their habitat are treated with a herbicidally active amount of a derivative I as claimed in claim 1.

**6.** Use of a compound of the formula I as claimed in claim 1 as a herbicide.

**7.** A plant growth regulator containing a compound I as claimed in claim 1 and inert additives.

**8.** A method for regulating plant growth, wherein an amount, having a regulating effect, of a compound of the formula I as claimed in claim 1 is allowed to act on the seeds, the plants and/or their habitat.

**9.** Use of a compound of the formula I as claimed in claim 1 for regulating plant growth.

**Revendications**

**1.** Dérivés d'acide carboxylique de la formule générale I

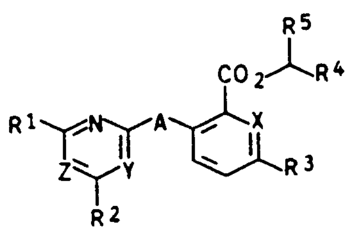

I,

dans laquelle les substituants ont la signification suivante :

$R^1$, $R^2$ alkyle en C1-C4, halogénalkyle en C1-C4, alcoxy en C1-C4, halogénalcoxy en C1-C4 ou alkyle en C1-C4-thio

$R^3$ hydrogène; hydroxy; cyano; nitro; amino; formyle; halogène, un groupe alkyle en C1-C4, alcoxy en C1-C4, alcényle en C3-C6, alcényloxy en C3-C6, alcinyle en C3-C6 ou alcinyloxy en C3-C6, ces groupes pouvant porter un à cinq atomes d'halogène et/ou un groupe alcoxy en C1-C4 ou alkyle en C1-C4-thio
un groupe alkyle en C1-C4-amino; di-alkyle en C1-C4-amino phénylamino; N-phényle-N-alkyle en C1-C4-amino ou alkyle enC1-C6-carbonylamino;

$R^4$ hydrogène ou alkyle en C1-C4

$R^5$ un reste hétéroaryle à 5 chaînons, qui contient un ou deux atomes d'azote et un atome d'oxygène ou de soufre, ou un reste d'isoxazolinyle, ces systèmes cyclique pouvant porter un à quatre atomes d'halogène et/ou un ou deux des restes suivants :
alkyle en C1-C6, halogénalkyle en C1-C2, alcoxy en C1-C4, halogénalcoxy en C1-C2, cycloalkyle en C3-C8, phényle ou pyridyle, les restes aromatiques de leur côté pouvant porter un à cinq atomes d'halogène et/ou un à trois des restes désignés par $R^1$
ou un groupe $-CR^6 = NOR^7$, où

$R^6$ désigne hydrogène, alkyle en C1-C4, qui peut porter un à cinq atomes d'halogène et/ou un des restes suivants :
alcoxy en C1-C4, (alkyle en C1-C4)-thio ou phényle, le reste phényle, de son côté, pouvant porter un à cinq atomes d'halogènes et/ou un à trois des restes désignés par $R^1$ ou être mis pour cycloalkyle en C3-C8 qui peut porter un à trois groupes alkyle en C1-C4;

phényle qui peut porter un à cinq atomes d'halogène et/ou un à trois des restes désignés par $R^1$, et

$R^7$ désigne alkyle en C1-C8 ou alcényle en C3-C6, qui peut porter un à cinq atomes d'halogène et/ou un des restes suivants : alcoxy en C1-C4, (alkyle en C1-C4)-thio, ou phényle, le reste phényle, de son côté, pouvant porter un à cinq atomes d'halogène et/ou un à trois des restes désignés par $R^1$, cycloalkyle en C3-C8 qui peut porter un à trois groupes alkyle en C1-C4, phényle qui peut porter un à cinq atomes d'halogène et/ou un à trois des restes désignés par $R^1$

A représente oxygène ou soufre

X représente azote ou un groupe méthone = $CR^8$, où

$R^8$ représente un des restes $R^3$ ou $R^8$ et $R^3$ ensemble représentant une chaîne 1,3-butadène-1,4-diyle ou une chaîne aza-1,3-butadiène-1,4-diyle, ces chaînes de leur côté pouvant porter un à quatre atomes d'halogène et/ou un ou deux des restes désignés par $R^1$,

Y,Z représentent azote ou un groupe méthine = CH-

ainsi que leurs sels utilisables en agriculture.

**2.** Procédé de préparation des composés I selon la revendication 1, caractérisé par le fait que l'on fait agir un dérivé d'acide carboxylique aromatique de la formule II

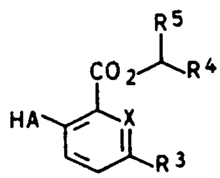

II

de manière connue en soi, en présence d'une base, avec un hétéroaromate de la formule III

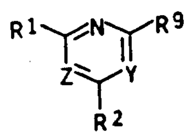

III

dans laquelle $R^9$ est mis pour un groupe éliminable.

**3.** Procédé de préparation des composés I selon la revendication 1, caractérisé par le fait que, on transforme d'abord, de manière connue en soi, un acide carboxylique de la formule IV

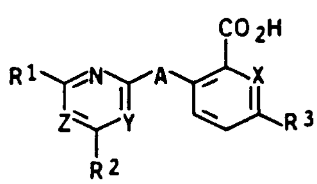

IV

en l'halogénure ou une autre forme activée de l'acide carboxylique et on estérifie ensuite ce dérivé avec un alcool substitué de la formule V

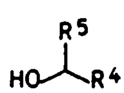

V

4. Herbicide contenant un composé de formule I selon la revendication 1 ainsi que des additifs inertes.

5. Procédé pour lutter contre la croissance des plantes indésirables, caractérisé par le fait qu'on traite les plantes indésirables et/ou leur biotope avec une quantité à activité herbicide d'un dérivé I selon la revendication 1.

6. Utilisation des composés de formule I selon la revendication 1 comme herbicide.

7. Moyen de régularisation de la croissance des plantes, contenant un composé I selon la revendication 1 ainsi que les additifs inertes.

8. Procédé de régularisation de la croissance des plantes, caractérisé par le fait qu'on fait agir une quantité à activité régulatrice d'un composé de formule I selon la revendication 2 sur les semences, les plantes et/ou leur biotope.

9. Utilisation de composés de formule I selon la revendication 1 pour la régularisation de la croissance des plantes.